# EUROPÄISCHE PATENTSCHRIFT

(11) **EP 2 846 725 B1**
(45) Veröffentlichungstag und Bekanntmachung des Hinweises auf die Patenterteilung: **22.04.2020**
(21) Anmeldenummer: 13719122.7
(22) Anmeldetag: 30.04.2013
(51) Int. Cl.: A61B 34/30, A61B 17/00, A61B 17/062

(54) **MINIMALINVASIVES INSTRUMENT FÜR DIE ROBOTISCHE CHIRURGIE**
MINIMALLY INVASIVE INSTRUMENT FOR ROBOTIC SURGERY
INSTRUMENT MINIMALEMENT INVASIF POUR LA CHIRURGIE ROBOTIQUE

(30) Priorität: 09.05.2012 DE 102012207707; 10.10.2012 US 201261711891 P
(43) Veröffentlichungstag der Anmeldung: 18.03.2015
(73) Patentinhaber: Deutsches Zentrum für Luft- und Raumfahrt e.V., 51147 Köln (DE)
(72) Erfinder: HAGN, Ulrich, 81543 München (DE); PASSIG, Georg, 85092 Kosching (DE); LANTERMANN (GEB. THIELMANN), Sophie, 81679 München (DE); FRÖHLICH, Florian, 82110 Germering (DE); SEIBOLD, Ulrich, Burnaby, BC V3N 4Z4 (CA)
(74) Vertreter: dompatent von Kreisler Selting Werner - Partnerschaft von Patent- und Rechtsanwälten mbB
(86) Internationale Anmeldenummer: PCT/EP2013/058996
(87) Internationale Veröffentlichungsnummer: WO 2013/167427

(56) Entgegenhaltungen:
- EP-A2- 1 779 801
- KR-A- 20110 003 229
- US-A1- 2007 016 174

## Beschreibung

Die Erfindung betrifft ein minimalinvasives Instrument für die robotische Chirurgie.

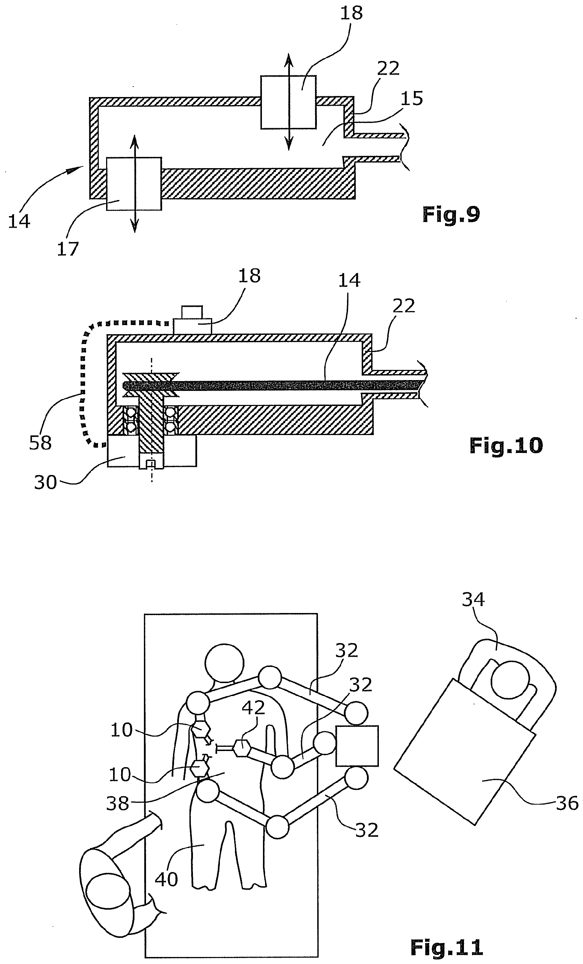

In der minimalinvasiven, robotergestützten Telechirurgie werden endoskopische Manipulationsinstrumente, z. B. Greifer, Nadelhalter usw. an Roboterarmen betrieben. Ein beispielhaftes Szenario ist in Figur 11 dargestellt. Die Roboterarme 32 werden vom Chirurgen 34 von einer Eingabestation 36 aus ferngesteuert. Dabei sind die endoskopischen Instrumente 10 an dem außerhalb des Patientenkörpers befindlichen Roboterarm 32 befestigt und gleichzeitig durch einen Zugang, beispielsweise einen Trokar 38 in der Körperhöhle des Patienten 40 in den Patientenkörper eingeführt. Die tatsächliche Operation findet im Körperinneren statt. Hierbei weisen die endoskopischen Instrumente 10 in Figur 11 nicht sichtbare funktionelle Instrumentenspitzen, wie z. B. Scheren, Zangen, Nadelhalter usw. auf. In der vorliegenden Patentanmeldung werden diese Instrumentenspitzen als Funktionselemente bezeichnet.

Vorteilhaft an dieser Operationsmethode ist, dass ein geringeres Trauma beim Patienten verursacht wird, da lediglich kleine Zugänge zum Patientenkörper notwendig sind. Es ist somit wünschenswert neben der Größe der Zugänge auch deren Anzahl zu reduzieren, um das Patiententrauma gering zu halten. Im Allgemeinen wird ein Zugang für das Instrument 10 der rechten Hand, ein Zugang für das Instrument 10 der linken Hand und ein Zugang für die Optik 42 (Endoskop) benötigt. Je nach Szenario können weitere, oder auch weniger Zugänge notwendig sein.

Während einer Operation wird im Körperinneren zusätzliches Material benötigt. Beispielsweise wird häufig Nahtmaterial, nämlich eine chirurgische Nadel mit Faden aber auch Klemmen, Tupfer etc. benötigt.

In der oben beschriebenen Konfiguration, die z. B. drei Zugänge zum Patientenkörper aufweisen kann, muss das besagte Material durch einen dieser drei Zugänge eingebracht bzw. wieder entfernt werden. Wenn das Legen eines zusätzlichen Zugangs vermieden werden soll, muss hierfür mindestens eines der Instrumente aus seinem Zugang entfernt werden.

In der robotischen minimalinvasiven Chirurgie werden spezielle minimalinvasive Instrumente an Roboterarmen verwendet, welche im Unterschied zu manuell betätigten minimalinvasiven Instrumenten (händische minimalinvasive Chirurgie) keinen Handgriff aufweisen, an dem sie durch einen Chirurgen gegriffen werden können oder mit welchem sie durch den Chirurgen betätigt werden können. Die Freiheitsgrade der robotischen/ angetriebenen Instrumente, beispielsweise eines Greifers werden durch Antriebe z. B. Elektromotoren im Instrument selbst oder im Roboterarm angetrieben. Bei der letzten Variante wird hierzu das Drehmoment der Antriebe am Roboterflansch zur Verfügung gestellt und vom Instrument mit einer Kupplung abgegriffen und auf die Freiheitsgrade des Instruments übertragen.

Systeme zum Einsatz in der minimalinvasiven robotergestützten Chirurgie sind in folgenden Veröffentlichungen beschrieben:
- S. Thielmann et.al. "MICA - A new generation of versatile instruments in robotic surgery," Proc. IROS, 2010,
- R. Devengenzo et.al. "Instrument interface of a robotic surgical system," US 2007/0119274 A1, 2007

Sofern bei Systemen, die aus dem Stand der Technik bekannt sind, während einer Operation chirurgisches Material, z. B. Nahtmaterial in den Patientenkörper eingebracht werden soll und hierfür kein zusätzlicher Zugang im Patientenkörper eröffnet werden soll, muss die Zureichung durch einen bestehenden Zugang geschehen. Eines der robotischen Instrumente muss somit entfernt werden. Nach dem Abkoppeln des Instruments vom robotischen System sind die Freiheitsgrade des Instruments entweder überhaupt nicht mehr ansteuerbar oder betätigbar (sofern die Betätigung durch einen Antrieb im Roboter erfolgte) oder nur durch den entfernt sitzenden Chirurgen ansteuerbar (sofern das Instrument einen eigenen Antrieb aufweist). Die Freiheitsgrade sind je nach System entweder blockiert oder vollkommen frei. Der Instrumentenwechsel während einer Operation wird jedoch üblicherweise durch Hilfspersonal, beispielsweise eine OP-Schwester durchgeführt. Solches Hilfspersonal kann mit einem derartigen Instrument nicht außerhalb des Patienten das Nahtmaterial greifen und mitsamt Instrument wieder in den Patienten einbringen.

In der aktuellen klinischen Prozedur wird deshalb zuerst das robotische Instrument entfernt. Die Hilfsperson greift mit einem manuellen minimalinvasiven Instrument das chirurgische Material außerhalb des Patienten und bringt das chirurgische Material mit dem manuellen Instrument in den Patientenkörper ein. Das manuelle Instrument wird nun entfernt und abschließend das robotische Instrument wieder eingeführt und am Roboter befestigt. Diese Vorgehensweise ist sehr zeitintensiv und aufwändig. Die gesamte Prozedur muss unter Kontrolle und Aufsicht des Chirurgen erfolgen, der jedes Zuführen und Entfernen mit dem Endoskop verfolgt, um das unbeaufsichtigte Verlieren von Gegenständen im Körper auszuschließen.

KR 2011-003229 beschreibt ein Robotersystem für die minimalinvasive Chirurgie. Zusätzlich zu einer Bedienung über die Eingabevorrichtung kann das Instrument auch über einen Griff bedient werden, der dem Griff eines konventionellen Instruments nachempfunden ist.

EP 1 779 801 A2 beschreibt ein manuelles minimalinvasives Instrument, das mittels einer Rastvorrichtung in verschiedenen Positionen arretiert werden kann.

Aufgabe der Erfindung ist es, ein minimalinvasives Instrument für die robotische Chirurgie bereitzustellen, durch das chirurgisches Material einfacher und schneller in den Patientenkörper eingeführt werden kann.

Die Lösung der Aufgabe erfolgt erfindungsgemäß durch die Merkmale des Anspruchs 1.

Das erfindungsgemäße minimalinvasive Instrument für die robotische Chirurgie weist ein Funktionselement wie beispielsweise einen Greifer und/ oder einen Nadelhalter auf.

Der Antrieb, mit dem das Funktionselement angetrieben wird, kann ein externer Antrieb sein, der außerhalb des minimalinvasiven Instruments angeordnet ist. Alternativ kann es sich um einen Antrieb handeln, der im oder am minimalinvasiven Instrument selbst angeordnet ist.

Das erfindungsgemäße Instrument weist ferner eine Kraft-, Drehmoment- und/ oder Druckübertragungsvorrichtung zum Übertragen von Kraft, Drehmoment und/ oder Druck von einem Antrieb zum Funktionselement auf. Bei einer Kraftübertragungsvorrichtung kann es sich beispielsweise um einen Seilzug oder eine Koppelstange handeln. Das Drehmoment kann in bekannter Weise durch Zahnräder, Torsionsstangen etc. übertragen werden. Wird eine Druckübertragungsvorrichtung verwendet, so wird ein Druck hydraulisch oder pneumatisch durch ein Fluid vom Antrieb zum Funktionselement übertragen und dort wieder zu einer Kraft und/ oder einem Drehmoment umgewandelt.

Das erfindungsgemäße Instrument weist ferner eine Koppelvorrichtung zum Koppeln des Instruments an einem medizinischen Roboter auf. Dies erfolgt insbesondere derart, dass in gekoppeltem Zustand das Funktionselement durch den Antrieb betätigbar ist.

Das erfindungsgemäße Instrument ist gekennzeichnet durch ein Bedienelement zum manuellen Bedienen des Funktionselements in einem Zustand, in dem das Instrument vom medizinischen Roboter abgekoppelt ist.

Durch das erfindungsgemäße Bedienelement kann somit das Funktionselement manuell, beispielsweise durch Hilfspersonal im Operationssaal bedient werden, während das minimalinvasive Instrument vom medizinischen Roboter abgekoppelt ist. Eine OP-Schwester kann somit zum Einführen chirurgischen Materials in den Patientenkörper das minimalinvasive Instrument vom medizinischen Roboter trennen, dieses aus dem Patientenkörper entfernen und mittels des manuell bedienbaren Bedienelements das chirurgische Material greifen und in den Körper des Patienten einbringen. Anschließend wird das minimalinvasive Instrument mit dem Roboter verbunden, so dass die Operation durch den Chirurgen fortgeführt werden kann.

Das Einbringen von chirurgischem Material in den Patientenkörper kann somit erfindungsgemäß wesentlich schneller und einfacher durchgeführt werden ohne einen Zwischenschritt über ein konventionelles manuelles Werkzeug.

Das Bedienelement kann auch dazu verwendet werden, innerhalb des Körpers des Patienten einen Gegenstand zu greifen (beispielsweise einen verbrauchten Tupfer oder eine Nadel), während das Instrument mit dem medizinischen Roboter gekoppelt ist. Hierzu kann z. B. der Chirurg unter Verwendung seiner Steuervorrichtung den zu entfernenden Gegenstand greifen, wonach das Bedienelement beispielsweise durch eine Hilfsperson manuell betätigt wird. Das Instrument kann dann vom Roboter abgekoppelt und aus dem Körper des Patienten entfernt werden.

Das Bedienelement kann beispielsweise zum Betätigen des Funktionselements auf die Kraft-, Drehmoment- und/ oder Druckübertragungsvorrichtung wirken. Verschiedene Varianten dieser Ausführungsform werden im weiteren Verlauf der vorliegenden Anmeldung beschrieben.

In einer alternativen Ausführungsform kann das erfindungsgemäße Instrument ein Getriebe zum Übertragen von Kräften und/ oder Drehmomenten vom Antrieb zum Funktionselement aufweisen. Das Bedienelement kann hierbei zum Betätigen des Funktionselements auf das Getriebe wirken. Auch diese Ausführungsform wird im weiteren Verlauf der vorliegenden Patentanmeldung näher beschrieben.

Das Bedienelement ist ein Hebel, der um einen fix mit dem Instrument verbundenen Schwenkpunkt schwenkbar ist. Der Hebel ist an einem Anlenkpunkt, der entlang des Hebels vom Schwenkpunkt beabstandet ist, mechanisch mit der Kraft-, Drehmoment- und/ oder Druckübertragungsvorrichtung koppelbar, so dass das Funktionselement betätigt werden kann.

Exemplarisch kann das Bedienelement anstelle eines Hebels linear verschiebbar sein. Auch dieses linear verschiebbare Bedienelement ist mechanisch mit der Kraft-, Drehmoment- und/ oder Druckübertragungsvorrichtung koppelbar.

Ein Rastelement zum Halten des Bedienelements in einer durch einen Nutzer vorgegebenen Position ist vorgesehen. Das Halten erfolgt hierbei derart, dass der Nutzer hierfür keine Kraft auf das Bedienelement aufbringen muss.

In einem weiteren Beispiel, das nicht Teil der Erfindung ist, ist das Bedienelement ein manuell betätigbares Handrad, das zum Betätigen des Funktionselements auf das Getriebe wirkt.

In einem anderen Beispiel ist das Bedienelement ein manuell betätigbarer Kolben, der zum Betätigen des Funktionselements auf die Druckübertragungsvorrichtung wirkt. Diese ist zur Druckübertragung vom Antrieb auf das Funktionselement mit einem Fluid gefüllt.

Bei den bisher beschriebenen Ausführungsformen muss das minimalinvasive Instrument selbst keinen eigenen Antrieb zum Betätigen des Funktionselements aufweisen. Vielmehr kann dieses durch einen externen Antrieb im medizinischen Roboter betätigt werden, der über die Koppelvorrichtung mit dem minimalinvasiven Instrument koppelbar ist.

In einer weiteren alternativen Ausführungsform ist das Bedienelement ein elektrisches und/ oder elektronisches Bedienelement, durch das ein elektrisches Signal erzeugbar ist. Das minimalinvasive Instrument weist hierbei einen eigenen internen Antrieb auf, dem das Signal des elektrischen und/ oder elektronischen Bedienelements zuführbar ist, so dass der interne Antrieb zum Betätigen des Funktionselements auf das Getriebe und/ oder die Kraft-, Drehmoment- und/ oder Druckübertragungsvorrichtung wirkt. Diese Ausführungsform ist besonders vorteilhaft, wenn das minimalinvasive Instrument ohnehin einen eigenen Antrieb aufweist. Dieser kann dazu verwendet werden, von dem elektronischen und/ oder elektrischen Bedienelement betätigt zu werden, wenn das minimalinvasive Instrument vom Roboter entkoppelt ist. Auch in dieser Ausführungsform kann somit das Funktionselement autark und manuell am minimalinvasiven Instrument selbst durch eine Hilfsperson betätigt werden.

Weiterhin ist es möglich, dass das Bedienelement eine eigene Kraft-, Drehmoment- und/ oder Druckübertragungsvorrichtung aufweist, über die das Funktionselement betätigt wird. Das Bedienelement greift somit direkt auf das Funktionselement zu. Dies kann beispielsweise bei einem seilbetriebenen Greifer der Fall sein.

Bei einer besonders bevorzugten Ausführungsform wird das Instrument durch einen internen Antrieb angetrieben. Zusätzlich ist das Funktionselement durch ein Bedienelement betätigbar, das von einem Benutzer direkt manuell betätigt wird. Hierbei kann es sich beispielsweise um einen mechanischen Hebel oder Ähnliches handeln. Das Bedienelement wirkt somit in dieser Ausführungsform beispielsweise nicht auf einen Elektromotor, durch den das Funktionselement betätigt wird. Vielmehr wird das Funktionselement ausschließlich mechanisch durch das Bedienelement betätigt. Der interne Antrieb des Instruments bzw. das mit diesem verbundene Getriebe ist hierbei durch die manuelle Betätigung des Bedienelements rücktreibbar und zwar insbesondere in einem Zustand, in dem das Instrument vom Roboter abgekoppelt ist.

In einer weiteren bevorzugten Ausführungsform ist das Bedienelement derart mit dem Funktionselement verbunden, dass eine Betätigung des Funktionselements durch das Bedienelement auch dann möglich ist, wenn das Instrument am Roboter befestigt ist. Dabei ist das Bedienelement derart an die Instrumentenmechanik angebunden, das es gegenüber den Steuereingaben eines Chirurgen Priorität besitzt. Somit kann beispielsweise eine Hilfskraft manuell zu jedem Zeitpunkt das Greifen von Nahtmaterial auslösen, was den Übergang zwischen Greifen durch den Chirurgen sowie dem Greifen und dem Entnehmen des Instruments durch den Assistenten erleichtert.

Im Folgenden werden bevorzugte Ausführungsformen der Erfindung anhand von Figuren erläutert.

Es zeigen:
- Figur 1: eine Draufsicht auf ein minimalinvasives Instrument,
- Figuren 2 bis 10: teilweise geschnittene Ansichten von minimalinvasiven Instrumenten,
- Figur 11: ein beispielhaftes Szenario für die robotische minimalinvasive Chirurgie.

Das in Figur 11 dargestellte beispielhafte Szenario für die robotische minimalinvasive Chirurgie wurde bereits im Zusammenhang mit dem Stand der Technik in der Beschreibungseinleitung erläutert.

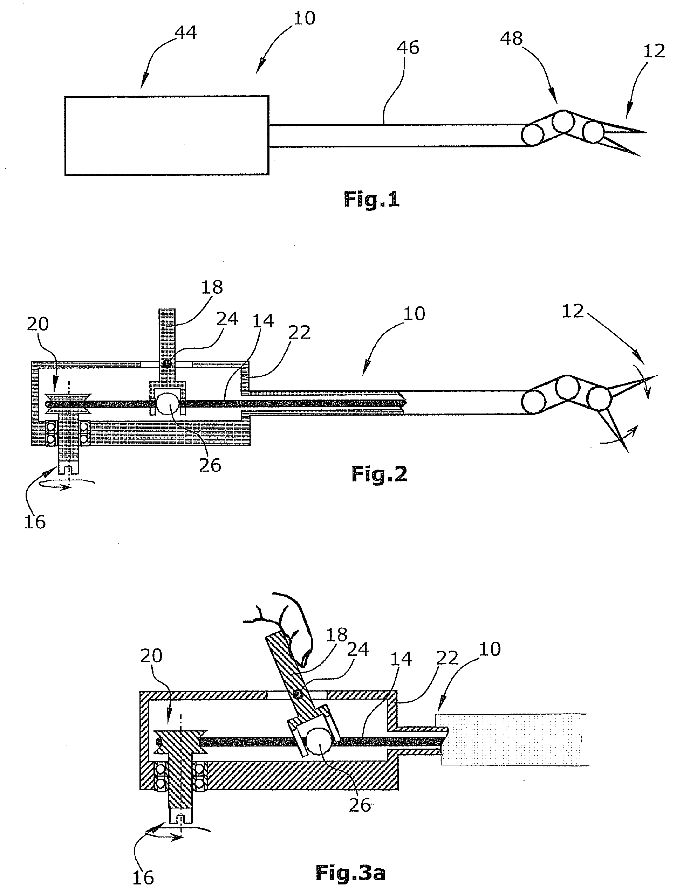

In Figur 1 ist ein minimalinvasives Instrument 10 mit einer Getriebeeinheit 44 dargestellt, die über einen Schaft 46 und Gelenke 48 mit dem Funktionselement 12 verbunden ist.

Ein beispielhafter interner Aufbau eines chirurgischen Instruments 10 ist in Figur 2 dargestellt. Das chirurgische Instrument 10 ist über die Koppelvorrichtung 16 mit einem externen Antrieb eines medizinischen Roboters verbindbar. Das übertragene Drehmoment wird mittels eines Getriebes 20, das beispielsweise mit einem Seilzug 14 mit dem Funktionselement 12 verbunden ist, an das Funktionselement 12 übertragen. Sofern das Instrument 10 vom medizinischen Roboter entkoppelt ist, kann das Funktionselement 12 nicht mehr durch den externen Antrieb betätigt werden. In diesem Zustand ist ein manuelles Betätigen des Funktionselements 12 durch das Bedienelement 18 möglich.

Dieses ist als Hebel ausgebildet, der um den Schwenkpunkt 24 im Gehäuse 22 des Instruments 10 verschwenkt werden kann. Über den Anlenkpunkt 26 entlang des Hebels 18 vom Schwenkpunkt 24 beabstandet, ist das Bedienelement 18 mit der Kraftübertragungsvorrichtung 14 gekoppelt.

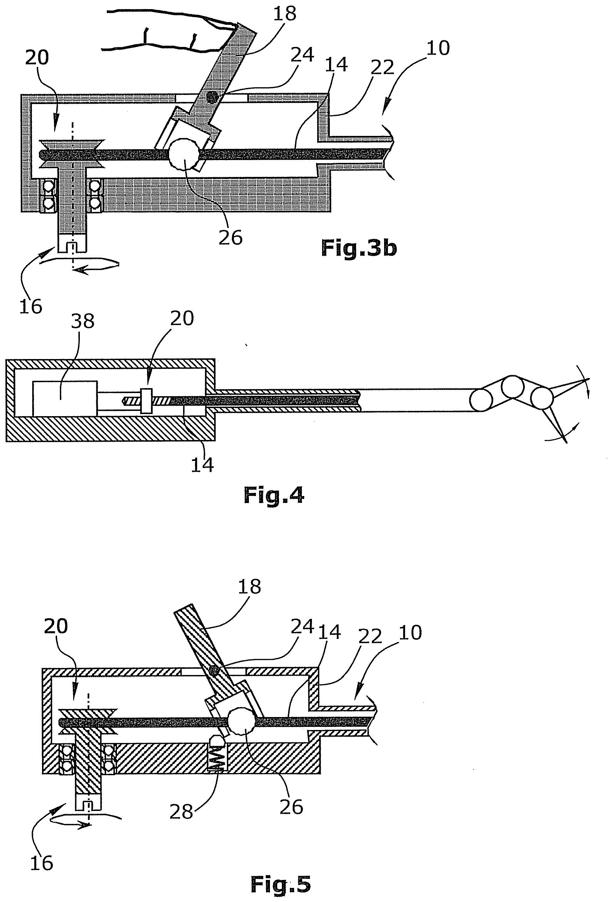

Die Betätigung eines solchen Hebels 18 durch eine Hilfsperson ist in Figuren 3a und 3b dargestellt. Das manuelle Bedienelement 18 stellt somit einen Bypass zum Antrieb des Freiheitsgrades des Funktionselements 12 durch den externen Antrieb dar. Der Hebel 18 ist hierbei am Anlenkpunkt 26 über ein Kugelterminal mit dem Kraftübertragungselement 14 mechanisch gekoppelt.

Das in Figur 4 dargestellte minimalinvasive Instrument 10 weist einen internen Antrieb 30 auf, der über ein Getriebe 20 und die Kraftübertragungsvorrichtung 14 mit dem Funktionselement 12 verbunden ist. Wie in Figur 10 dargestellt, kann diese Ausführungsform dazu verwendet werden, den internen Antrieb 30 durch ein elektrisches und/ oder elektronisches Bedienelement 18 zu betätigen, so dass das Funktionselement 12 auch betätigt werden kann, wenn das Instrument 10 vom Roboter entkoppelt ist. Das Instrument 10 kann auch zwei interne Antriebe aufweisen, wobei ein Antrieb das Funktionselement 12 antreibt, wenn das Instrument 10 mit dem Roboter gekoppelt ist und der zweite Antrieb zum Betätigen des Funktionselements 12 im endkoppelten Zustand dient.

Alternative Ausführungsformen des erfindungsgemäßen Bedienelements 18 sind in den Figuren 5 bis 9 dargestellt:
In Figur 5 ist das Bedienelement 18 wie der bereits beschriebene Hebel ausgebildet. An einer geeigneten Stelle, z. B. der Unterseite des Gehäuses 22 des Instruments 10 ist ein Rastelement 28 vorgesehen, das beispielsweise eine federbelastete Kugel sein kann. Die Federkraft wirkt hierbei in Richtung des Bedienelements 18, so dass das Rastelement das Bedienelement in einer durch den Nutzer gewünschten Stellung hält, ohne dass der Nutzer eine Kraft auf das Bedienelement 18 aufbringen muss.

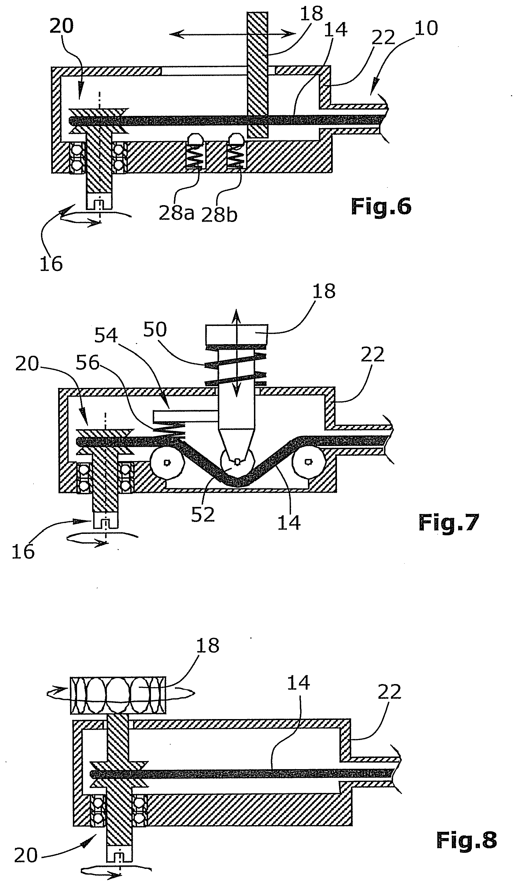

In Figur 6 ist ein linear verschiebbares Bedienelement 18 dargestellt, das mit zwei Rastvorrichtungen 28a, 28b zusammenwirken kann. Das Bedienelement 18 kann somit an drei verschiedenen Positionen gehalten werden.

In einer alternativen Ausführungsform kann die Rastvorrichtung durch eine Schaltschablone ausgebildet sein, in der ein linear verschiebbarer Hebel 18 verschoben wird. Diese Schaltschablone kann mehrere Rastpositionen zum Einrasten des Hebels 18 aufweisen. Zwischen diesen Rastpositionen kann sich der Hebel frei bewegen.

Das in Figur 7 dargestellte Bedienelement 18 ist ebenfalls linear verschiebbar. Diese lineare Verschiebung erfolgt allerdings in einer im Wesentlichen senkrechten Richtung relativ zur Verlaufsrichtung des Kraftübertragungselements 14. Das Bedienelement 18 stützt sich z. B. mit einer Feder 50 z. B. am Gehäuse 22 ab. Am unteren Ende weist das Bedienelement 18 vorzugsweise eine Rolle 52 auf, die auf die Kraftübertragungsvorrichtung 14 wirkt. Ist die Kraftübertragungsvorrichtung 14 durch ein oder mehrere Seile ausgebildet, kann die Rolle 52 auch auf mehrere Seile gleichzeitig wirken, um das Funktionselement zu betätigen. Weiterhin weist dieses Bedienelement 18 vorzugsweise eine Klemmvorrichtung 54 auf, die mittels einer Feder 56 das getriebeseitige Ende der Kraftübertragungsvorrichtung 14, beispielsweise eines Seils festlegt, so dass sich die Betätigung des Bedienelements 18 ausschließlich auf das schaftseitige Seilende auswirkt.

In Figur 8 ist das Bedienelement 18 als ein Handrad ausgebildet, das direkt auf das Getriebe 20 wirkt. Zum Öffnen und Schließen des Funktionselements 12 wird somit das Handrad 18 gedreht.

In Figur 9 wird die Kraft von einem externen Antrieb über die Druckübertragungsvorrichtung 14 zum nicht dargestellten Funktionselement 12 übertragen. Hierfür ist ein erster Kolben 17 vorgesehen, der auf das Fluid 15 im Inneren des Gehäuses 22 wirkt. Dieses wiederum erzeugt einen Druck auf ein nicht dargestelltes Element, das mit dem Funktionselement 12 gekoppelt ist. Beim Abkoppeln des Instruments 10 vom medizinischen Roboter muss hierbei der erste Kolben 17 blockiert werden, so dass durch das Bedienelement 18, das ebenfalls als Kolben ausgebildet ist, der auf das Fluid 15 wirkt, das Funktionselement 12 manuell betätigt werden kann.

Die Ausführungsform gemäß Figur 10 wurde bereits im Zusammenhang mit Figur 4 erläutert. Das hier dargestellte elektrische und/ oder elektronische Bedienelement 18 kann ein Schalter, ein Taster, ein Joystick, ein Potentiometer, ein Scrollrad usw. sein, das über eine Signalverbindung 58 mit dem internen Antrieb 30 verbunden ist. In dieser Ausführungsform kann das Instrument 10 auch eine Energieversorgung, beispielsweise eine Batterie, ein Akku oder ein Kondensator etc. aufweisen, um die Instrumentenfunktion auch im vom Roboter abgekoppelten Zustand zumindest kurzzeitig zu gewährleisten.

## Patentansprüche

1. Minimalinvasives Instrument für die robotische Chirurgie,
mit einem Funktionselement (12),
einer Kraft-, Drehmoment- und/ oder Druckübertragungsvorrichtung (14) zum Übertragen von Kraft, Drehmoment und/ oder Druck von einem Antrieb zum Funktionselement (12),
einer Koppelvorrichtung (16) zum Koppeln des Instruments (10) an einen medizinischen Roboter, insbesondere derart, dass das Funktionselement (12) durch den Antrieb betätigbar ist,
einem Bedienelement (18) zum manuellen Bedienen des Funktionselements (12) in einem Zustand, in dem das Instrument (10) vom medizinischen Roboter abgekoppelt ist,
**gekennzeichnet durch** ein Rastelement (28, 28a, 28b) zum Halten des Bedienelements (18) in einer durch einen Nutzer vorgegebenen Position, ohne dass der Nutzer hierfür eine Kraft auf das Bedienelement (18) aufbringen muss, wobei das Bedienelement (18) ein Hebel ist, der um einen mit dem Instrument (10) verbundenen Schwenkpunkt (24) schwenkbar ist und der Hebel (18) an einem Anlenkpunkt (26), der vom Schwenkpunkt (24) beabstandet ist, mechanisch mit der Kraft-, Drehmoment- und/ oder Druckübertragungsvorrichtung (14) koppelbar ist.

2. Instrument nach Anspruch 1, **dadurch gekennzeichnet, dass** das Funktionselement (12) ein Greifer, ein Nadelhalter und/ oder eine Schere ist.

3. Instrument nach Anspruch 1 oder 2, **dadurch gekennzeichnet, dass** das Bedienelement (18) zum Betätigen des Funktionselements (12) auf die Kraft-, Drehmoment- und/ oder Druckübertragungsvorrichtung (14) wirkt oder eine separate Kraft-, Drehmoment- und/ oder Druckübertragungsvorrichtung (14) zum Funktionselement (12) aufweist.

4. Instrument nach Anspruch 1 oder 2, **gekennzeichnet durch** ein Getriebe (20) zum Übertragen von Kräften und/ oder Drehmomenten vom Antrieb zum Funktionselement (12), wobei das Bedienelement (18) zum Betätigen des Funktionselements (12) auf das Getriebe (20) wirkt.

5. Instrument nach Anspruch 3 oder 4, **dadurch gekennzeichnet, dass** das Bedienelement (18) linear verschiebbar und mechanisch mit der Kraft-, Drehmoment- und/ oder Druckübertragungsvorrichtung (14) koppelbar ist.

6. Instrument nach Anspruch 4, **dadurch gekennzeichnet, dass** das Bedienelement (18) ein manuell betätigbares Handrad ist, das zum Betätigen des Funktionselements (12) auf das Getriebe (20) oder die Kraft-, Drehmoment- und/ oder Druckübertragungsvorrichtung (14) wirkt.

7. Instrument nach einem der Ansprüche 1 bis 6, **dadurch gekennzeichnet, dass** das Bedienelement (18) ein manuell betätigbarer Kolben ist, der zum Betätigen des Funktionselements (12) auf die Druckübertragungsvorrichtung (14) wirkt, die zur Druckübertragung mit einem Fluid (15) gefüllt ist.

8. Instrument nach einem der Ansprüche 1 bis 7, **dadurch gekennzeichnet, dass** das Bedienelement (18) ein elektrisches und/ oder elektronisches Bedienelement ist, durch das ein elektrisches Signal erzeugbar ist, wobei das Instrument (10) einen internen Antrieb (30) aufweist, dem das Signal des elektrischen und/ oder elektronischen Bedienelements (18) zuführbar ist, so dass der interne Antrieb (30) zum Betätigen des Funktionselements (12) auf das Getriebe (20) und/ oder die Kraft-, Drehmoment- und/ oder Druckübertragungsvorrichtung (14) wirkt.

## Claims

1. A minimally invasive instrument for robotic surgery,
including a functional element (12),
a force, torque and/or pressure transmission device (14) for transmitting force, torque and/or pressure from a drive to said functional element (12),
a coupling device (16) for coupling the instrument (10) with a medical robot, in particular such that said functional element (12) is adapted to be actuated via the drive,
an operating element (18) for manually operating said functional element (12) in a state in which said instrument (10) is uncoupled from the medical robot,
**characterized by**
a latch element (28, 28a, 28b) for retaining the operating element (18) in a position determined by the user without the user having to apply a force upon said operating element (18),
wherein the operating element (18) is a lever which is adapted to be pivoted about a pivot point (24) connected with the instrument (10), and said lever (18) is adapted to be mechanically coupled with the force, torque and/or pressure transmission device (14) at a hinge point (26) which is spaced apart from said pivot point (24).

2. The instrument according to claim 1, **characterized in that** the functional element (12) is a gripper, a needle holder and/or scissors.

3. The instrument according to claim 1 or 2, **characterized in that** the operating element (18) acts upon the force, torque and/or pressure transmission device (14) for actuating the functional element (12) or comprises a separate force, torque and/or pressure transmission device (14) to said functional element (12).

4. The instrument according to claim 1 or 2, **characterized by** a gear (20) for transmitting forces and/or torques from the drive to the functional element (12), wherein the operating element (18) acts upon said gear (20) for actuating said functional element (12).

5. The instrument according to claim 3 or 4, **characterized in that** the operating element (18) is adapted to be linearly displaced and to be mechanically coupled with the force, torque and/or pressure transmission device (14).

6. The instrument according to claim 4, **characterized in that** the operating element (18) is a manually operable hand wheel which acts upon the gear (20) or the force, torque and/or pressure transmission device (14) for actuating the functional element (12).

7. The instrument according to any one of claims 1 to 6, **characterized in that** the operating element (18) is a manually operable plunger which acts upon the pressure transmission device (14) for actuating the functional element (12), said pressure transmission device being filled with a fluid (15) for pressure transmission purposes.

8. The instrument according to any one of claims 1 to 7, **characterized in that** the operating element (18) is an electric and/or electronic operating element which is adapted to generate an electric signal, wherein the instrument (10) comprises an internal drive (30) which is adapted to be supplied with the signal from said electric and/or electronic operating element (18) such that said internal drive (30) acts upon the gear (20) and/or the force, torque and/or pressure transmission device (14) for actuating the functional element (12).

## Revendications

1. Instrument minimalement invasif destiné à la chirurgie robotique,
doté d'un élément fonctionnel (12),
d'un dispositif de transmission de force, de couple ou de pression (14) permettant de transmettre une force, un couple et/ou une pression depuis un entraînement vers l'élément fonctionnel (12),
d'un dispositif de couplage (16) permettant de coupler l'instrument (10) à un robot médical, en particulier de sorte que l'élément fonctionnel (12) puisse être actionné par l'entraînement,
d'un élément de commande (18) permettant de commander manuellement l'élément fonctionnel (12) dans un état où l'instrument (10) est découplé du robot médical,
**caractérisé par** un élément de cliquet (28, 28a, 28b) permettant de maintenir l'élément de commande (18) dans une position prédéfinie par un utilisateur, sans que l'utilisateur ne doive exercer à cet effet une force sur l'élément de commande (18),
dans lequel l'élément de commande (18) est un levier, lequel peut pivoter autour d'un point de pivot (24) relié à l'instrument (10) et le levier (18) peut être couplé à un point d'articulation (26), lequel est écarté du point de pivot (24), mécaniquement avec le dispositif de transmission de force, de couple ou de pression (14).

2. Instrument selon la revendication 1, **caractérisé en ce que** l'élément fonctionnel (12) est une pince, un porte-aiguille et/ou une cisaille.

3. Instrument selon la revendication 1 ou 2, **caractérisé en ce que** l'élément de commande (18) permettant d'actionner l'élément fonctionnel (12) opère sur le dispositif de transmission de force, de couple ou de pression (14) et/ou comporte un dispositif séparé de transmission de force, de couple ou de pression (14) pour l'élément fonctionnel (12).

4. Instrument selon la revendication 1 ou 2, **caractérisé par** une transmission (20) permettant de communiquer des forces et/ou des couples depuis l'entraînement jusqu'à l'élément fonctionnel (12), dans lequel l'élément de commande (18) opère sur la transmission (20) afin d'actionner l'élément fonctionnel (12).

5. Instrument selon la revendication 3 ou 4, **caractérisé en ce que** l'élément de commande (18) peut coulisser de manière linéaire et peut être couplé mécaniquement au dispositif de transmission de force, de couple ou de pression (14).

6. Instrument selon la revendication 4, **caractérisé en ce que** l'élément de commande (18) est un volant manuellement actionnable, lequel opère sur la transmission (20) ou sur le dispositif de transmission de force, de couple ou de pression (14) afin d'actionner l'élément fonctionnel (12) .

7. Instrument selon l'une des revendications 1 à 6, **caractérisé en ce que** l'élément de commande (18) est un piston manuellement actionnable, lequel opère sur le dispositif de transmission de pression (14), lequel est rempli d'un fluide (15) destiné à communiquer la pression, afin d'actionner l'élément fonctionnel (12).

8. Instrument selon l'une des revendications 1 à 7, **caractérisé en ce que** l'élément de commande (18) est un élément de commande électrique et/ou électronique, par lequel un signal électrique peut être produit, dans lequel l'instrument (10) comporte un entraînement interne (30), auquel le signal de l'élément de commande électrique et/ou électronique (18) peut être envoyé, de sorte que l'entraînement interne (30) opère sur la transmission (20) ou sur le dispositif de transmission de force, de couple ou de pression (14) afin d'actionner l'élément fonctionnel (12).
